# EUROPEAN PATENT SPECIFICATION

(11) **EP 4 439 107 B1**
(45) Date of publication and mention of the grant of the patent: **19.08.2026**
(21) Application number: 24164186.9
(22) Date of filing: 18.03.2024
(51) Int. Cl.: G01R 33/567

(54) **MAGNETIC RESONANCE IMAGING APPARATUS AND CONTROL METHOD OF MAGNETIC RESONANCE IMAGING APPARATUS**
MAGNETRESONANZBILDGEBUNGSVORRICHTUNG UND STEUERUNGSVERFAHREN FÜR EINE MAGNETRESONANZBILDGEBUNGSVORRICHTUNG
APPAREIL D'IMAGERIE PAR RÉSONANCE MAGNÉTIQUE ET PROCÉDÉ DE COMMANDE D'APPAREIL D'IMAGERIE PAR RÉSONANCE MAGNÉTIQUE

(30) Priority: 27.03.2023 JP 2023050038
(43) Date of publication of application: 02.10.2024
(73) Proprietor: FUJIFILM Corporation, Tokyo 106-8620 (JP)
(72) Inventor: GOTO, Tomohiro, Kashiwa-shi, Chiba, 277-0804 (JP)
(74) Representative: Strehl & Partner mbB

(56) References cited:
- US-A1- 2009 062 640
- US-A1- 2010 264 922
- US-A1- 2015 173 642
- SINGH G ET AL: "Improved Signal-to-Noise Ratio in Parallel Coronary Artery Magnetic Resonance Angiography using Graph Cuts based Bayesian Reconstruction", CONFERENCE PROCEEDINGS. ANNUAL INTERNATIONAL CONFERENCE OF THE IEEE ENGINEERING IN MEDICINE AND BIOLOGY SOCIETY (IEEE CAT. NO. 06CH37748); 30 AUG.-3 SEPT. 2006; NEW YORK, NY, USA, IEEE, PISCATAWAY, NJ, USA, 30 August 2006 (2006-08-30), pages 703 - 706, XP031339305, ISBN: 978-1-4244-0032-4
- GILCHRIST STUART ET AL: "A simple, open and extensible gating Control unit for cardiac and respiratory synchronisation control in small animal MRI and demonstration of its robust performance in steady-state maintained CINE-MRI", MAGNETIC RESONANCE IMAGING, ELSEVIER SCIENCE, TARRYTOWN, NY, US, vol. 81, 24 April 2021 (2021-04-24), pages 1 - 9, XP086684742, ISSN: 0730-725X, [retrieved on 20210424], DOI: 10.1016/J.MRI.2021.04.012

## Description

The present application claims priority from Japanese patent application JP-2023-050038 filed on Mar 27, 2023.

### BACKGROUND OF THE INVENTION

### 1. Field of the Invention

The present invention relates to a magnetic resonance imaging apparatus (hereinafter, referred to as an MRI apparatus), and particularly relates to a control method of an MRI apparatus in a case of performing electrocardiogram gating imaging.

### 2. Description of the Related Art

In a case in which a target of an image diagnosis is a tissue such as a heart or a blood vessel around the heart by using the MRI apparatus, an image is affected by the movement of the heart and the respiratory movement, and a body movement artifact occurs. In order to suppress the body movement artifact, it is necessary to acquire image data at a timing at which the heartbeat and the respiratory movement are stable. In general, a nuclear magnetic resonance signal from a subject is acquired at a time phase (diastole) with a relatively small movement in a cardiac period and at a timing at which the respiratory movement is almost not performed in an expiration period (stable period), and an image in which an influence of the movement of the heart is eliminated as much as possible is obtained.

Therefore, in the related art, a signal from an electrocardiograph or a heart rate meter is received, and an imaging pulse sequence is executed in the diastole after a predetermined delay time with an occurrence time of an R wave or the like as a trigger. In this case, a navigation echo for respiratory movement monitoring (hereinafter, referred to as a navi echo) and main imaging are executed as a set, and an image reconstruction signal (image data) is collected only in a case in which a respiratory displacement is within a threshold value of a preset number of mm (JP2011-147560A).

US 2010/0264922 A1 discloses an MRI apparatus and method with the features in the preamble of present independent claims 1 and 7.

### SUMMARY OF THE INVENTION

In double gating in which the electrocardiogram gating and the respiratory movement gating in the related art are used in combination, data is collected depending on a respiratory period and a heartbeat period of the subject, and thus the data acquisition efficiency is low and an imaging time may be long. For example, there is imaging in which the data for two heartbeats is acquired in the stable period of expiration, and the data is acquired once in two heartbeats. However, in control of the related art, in a case in which the respiratory displacement after the R wave detection deviates from the set threshold value, the main imaging is not executed. Therefore, in the imaging in which the data is acquired in two heartbeats, the extension for two heartbeats is accumulated. In addition, in the subject having a short stable period of the respiratory movement, it is difficult to perform imaging for acquiring the data with two heartbeats.

An object of the present invention is to improve data acquisition efficiency in double gating imaging. Another object of the present invention is to suppress the influence of the body movement and enable the acquisition of data in two consecutive heartbeats even in a case in which the stable period of the respiratory movement of the subject is relatively short.

In order to solve the above-described problem, the present invention provides the apparatus defined in claim 1 and the method defined in claim 7. The start of a respiratory stable period (expiration) is detected by monitoring the respiratory movement during imaging. After receiving the electrocardiogram gating signal, in a case in which the respiratory movement immediately before the electrocardiogram gating signal is received is transitioned to the respiratory stable period, image data is acquired at a timing at which a cardiac time phase and a respiratory time phase of the subject are matched with each other. Accordingly, the image data for two heartbeats can be acquired in each respiratory period.

Generally, the present invention relates to an MRI apparatus comprising: an imaging unit that collects a nuclear magnetic resonance signal generated from a subject, and performs a navigation measurement for detecting a respiratory movement waveform of the subject and a main measurement for generating an image of the subject; a measurement controller that controls the imaging unit; a prior information setting unit that sets a gate window at a position in a stable period of the respiratory movement waveform of the subject; a respiratory movement monitoring unit that monitors the respiratory movement of the subject by using a result of the navigation measurement, and transmits an accept signal and a reject signal to the measurement controller depending on whether a magnitude of the respiratory movement of the subject is inside or outside a preset gate window; and a cardiac period monitoring unit that monitors a cardiac period of the subject, and generates a gate signal at an R wave occurrence timing of the cardiac period.

The measurement controller consecutively executes the navigation measurement before and after the generation of the gate signal, and controls, in a case in which the gate signal is received from the cardiac period monitoring unit, the imaging unit to perform the main measurement after a preset delay time elapses in a case in which a most recent transition among transitions of a signal from the respiratory movement monitoring unit, which is generated during the navigation measurement before the reception of the gate signal, is a transition from the reject signal to the accept signal. The prior information setting unit is configured to set an upper limit of the gate window to a value lower than a maximum value of the stable period of the respiratory movement waveform of the subject so that the transition from the reject signal to the accept signal occurs once before and after an apex of the respiratory movement waveform, respectively.

Even in a case in which the signal, which is most recent to the gate signal, from the respiratory movement monitoring unit is consecutively the accept signal twice or more, the main measurement is performed after the delay time.

The start of the main measurement is controlled by using the respiratory movement monitoring result immediately before the gate signal for performing the main measurement, whereby it is possible to ensure a period of small respiratory movement and to acquire the data at a fixed cardiac time phase. As a result, the body movement artifact can be suppressed. In addition, since the data can be efficiently acquired at a timing at which the cardiac time phase and the respiratory displacement are matched with each other, it is possible to minimize the extension of the imaging time. Further, since the displacement due to the respiratory movement in the acquisition of the image data can be detected from the navi echo in a delay period, an imaging slice position corresponding to the detected displacement can be corrected, and an image in which the misregistration is suppressed can be acquired.

### BRIEF DESCRIPTION OF THE DRAWINGS

Fig. 1 is a block diagram showing a configuration example of an MRI apparatus according to an embodiment of the present invention.
Fig. 2 is a block diagram including details of an imaging unit of the MRI apparatus.
Fig. 3 is a diagram showing a process of the MRI apparatus according to the embodiment of the present invention.
Fig. 4 is a diagram showing a process of prior information setting.
Fig. 5 is a diagram showing a flow of control of Embodiment 1 which does not fall under the scope of the claims and is not an embodiment of the invention.
Fig. 6 is a diagram showing control of Embodiment 1.
Fig. 7 is a diagram showing control of Embodiment 2 which is an embodiment of the invention.

### DESCRIPTION OF THE PREFERRED EMBODIMENTS

Hereinafter, an MRI apparatus and a control method of the MRI apparatus according to the embodiment of the present invention will be described.

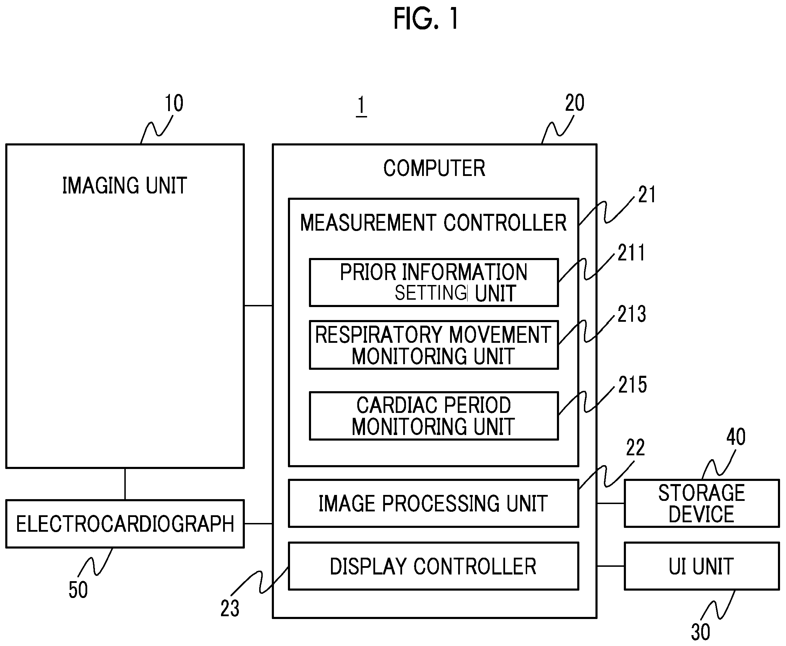

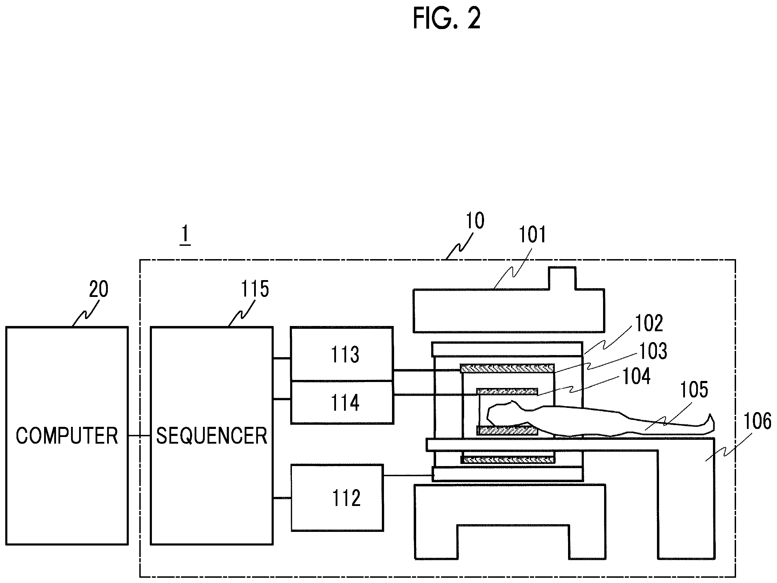

First, an overall configuration of the MRI apparatus will be described with reference to Figs. 1 and 2.

As shown in Fig. 1, an MRI apparatus 1 comprises an imaging unit 10 that collects a nuclear magnetic resonance signal generated from a subject, a computer 20 that controls the imaging unit 10 and performs an operation such as image reconstruction using the nuclear magnetic resonance signal collected by the imaging unit 10, a UI unit 30 comprising a display device, an input device, and the like, and a storage device 40.

The present invention is a technique applied to electrocardiogram gating imaging using the MRI apparatus, and has features in a configuration and a function of a controller of the MRI apparatus for controlling the gating imaging. In the computer 20 that functions as the controller, as functions of a measurement controller 21 that controls measurement, a respiratory movement monitoring unit 213 that monitors a respiratory movement using a navi echo and emits a signal necessary for measurement control, a cardiac period monitoring unit 215 that inputs a signal of an electrocardiograph 50 or the like attached to the subject and monitors a cardiac period, and a prior information setting unit 211 that sets a parameter necessary for the gating imaging are provided.

As in a general MRI apparatus, in addition to the measurement controller 21, the computer 20 comprises an image processing unit 22 that performs image reconstruction or other image processing by using the nuclear magnetic resonance signal collected from the subject, a display controller 23 that controls the display of the UI unit 30, and the like.

The functions of the computer 20 described above are realized by reading a program capable of realizing each function in a general-purpose computer or workstation, but a part of the functions can also be realized by hardware such as a programmable IC. Further, a part of the functions may be realized by a processing apparatus different from the MRI apparatus.

The configuration and the function of the imaging unit 10 are the same as those of the general MRI apparatus. Briefly, as shown in Fig. 2, the imaging unit 10 comprises a magnet 101 that generates a static magnetic field, a gradient magnetic field coil 102 that generates a gradient magnetic field in three axial directions orthogonal to each other, an RF transmission coil 103 that emits a high-frequency magnetic field, an RF reception coil 104 that detects the nuclear magnetic resonance signal generated from a subject 105, a transmitter 113 that transmits a predetermined high-frequency current to the RF transmission coil 103, a receiver 114 to which the RF reception coil 104 is connected, a gradient magnetic field power source 112 that drives the gradient magnetic field coil 102, and a sequencer 115. In a case in which one RF coil serves as both the RF transmission coil 103 and the RF reception coil 104, a switch (not shown) is added between the transmitter 113 and the receiver 114 in this case. The sequencer 115 drives the transmitter 113, the receiver 114, and the gradient magnetic field power source 112 in accordance with the pulse sequence under the control of the controller.

The subject 105 is normally transported into a static magnetic field space (imaging space) generated by the magnet 101 in a state of lying on a bed 106, and imaging is performed after an examination part is positioned at the center of the static magnetic field space.

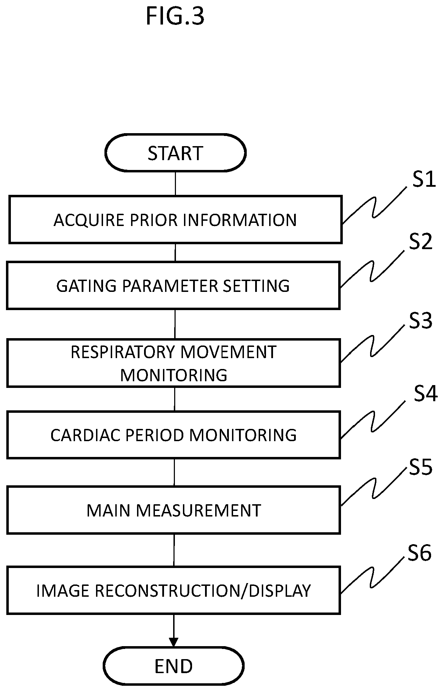

Next, the imaging in which double gating of the electrocardiogram gating and the respiratory movement gating are performed by using the MRI apparatus will be described based on the above configuration. An outline of the process will be described with reference to Fig. 3.

First, as pre-measurements, scout imaging for positioning the subject in the imaging space, scanogram (hereinafter, abbreviated to scano) for imaging three cross sections of the imaging part at the center in the imaging space, and heart cine imaging are performed (S1). Gating parameters necessary for controlling the main imaging are set based on these pre-measurements (S2). The gating parameters include a gate window for monitoring a stable period of the respiratory movement, a delay time from a specific time phase (R wave) of a cardiac period to a main measurement, and navi echo acquisition position. Since the gating parameters are different depending on the subject, the gating parameters are determined from an image or data obtained by the pre-measurement using the subject as a target.

Then, a navigation measurement is started, and the monitoring of the respiratory movement is started (S3). In addition, an electrocardiogram waveform from the electrocardiograph 50 is taken in at the same time as the navigation measurement or before and after the navigation measurement, and the cardiac period is monitored (S4).

The main measurement is executed at a timing at which the respiratory movement is in a predetermined stable period and the cardiac time phase is the specific time phase, and the image data is collected (S5). Therefore, the transition of the cardiac period acquired at a timing that is traced back from the specific time phase (R wave) detection (transition from outside the gate window to inside the gate window) is confirmed, and the execution of the main measurement is determined by the result.

The main measurement is, for example, heart imaging, and a known two-dimensional (2D) or three-dimensional (3D) pulse sequence is executed. In this case, the cardiac time phase at the time of the image data acquisition is fixed, and a slice position of the main measurement is corrected as necessary based on the displacement of the respiratory movement obtained from the navi echo acquired before the acquisition of the image data. After collecting the image data necessary for the image reconstruction, the image reconstruction and the display are performed (S6).

With the above process, it is possible to execute the imaging by using the timing at which the cardiac time phase and the respiratory displacement are matched with each other as much as possible, to improve the data acquisition efficiency, and to suppress the extension of the imaging time. In addition, since imaging can be performed without missing the timing at which the cardiac time phase and the respiratory displacement are matched with each other, it is also possible to perform imaging in which the main measurement is performed twice in each respiratory period.

Hereinafter, a specific embodiment of each process and the control thereof will be described with an example of a case in which an imaging target is the heart. Since the process of Fig. 3 is common to the following embodiments, the process of Fig. 3 will be appropriately described with reference to the following embodiments.

### Embodiment 1

Embodiment 1 is not an embodiment of the invention but an example in which the subject whose respiratory movement is relatively stable is a target, and a window for the respiratory movement monitoring is set so that the entire period (expiration period) in which the expiration of the respiratory movement having a relatively long duration is included.

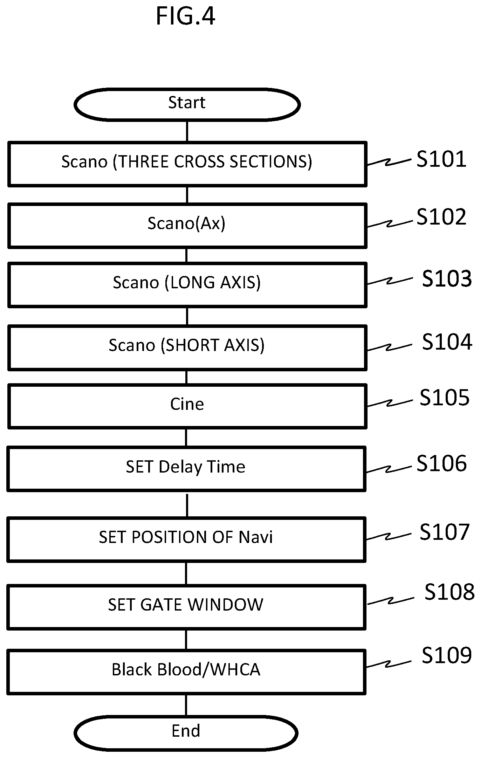

First, details of the pre-measurement (S1) and the gating parameter setting (S2) related to the prior information setting unit 211 will be described with reference to Fig. 4.

After positioning the imaging part of the subject, the scano is performed, and the imaging target part of the subject is imaged (S101 to S104). In the scano, for example, after first imaging the three cross sections (Ax, COR, SAG) with the heart as the center of the magnetic field (S101), the Ax cross section near the center of the left ventricle determined on the three cross sections is imaged (S102), a long axis cross section of the left ventricle determined on the Ax cross section of S102 is imaged (S103), and a short axis cross section of the left ventricle determined on the Ax cross section of S102 and the long axis cross section of S103 is imaged (S104).

Subsequently, a cine image is captured for the cross sections determined in S102 or S104 (S105), and the cine image is displayed on the display device of the UI unit 30. The cine image can capture the movement of the heart along a time axis by consecutive imaging with a relatively low resolution. A user can confirm a start timing of the period (diastole) in which the pulsation is small by looking at the cine image displayed on the display device, and sets the start timing to the delay time of the imaging performed thereafter (S106).

Next, the user sets a part at a predetermined position from the heart as a position for acquiring the navi echo (S107). The position of the navi echo is not limited, but is usually set on a diaphragm. The position of the diaphragm can be set, for example, via the UI unit 30 based on an SAG image.

Then, the gate window for the navigation measurement is determined (S108). For example, the gate window position is matched with an upper end of the diaphragm on the SAG image obtained in S101. Accordingly, it is possible to collect the data within the expiration period in which the respiratory movement is stable. A gate window width is set to a predetermined value, for example, about 3 mm in advance as a scan parameter.

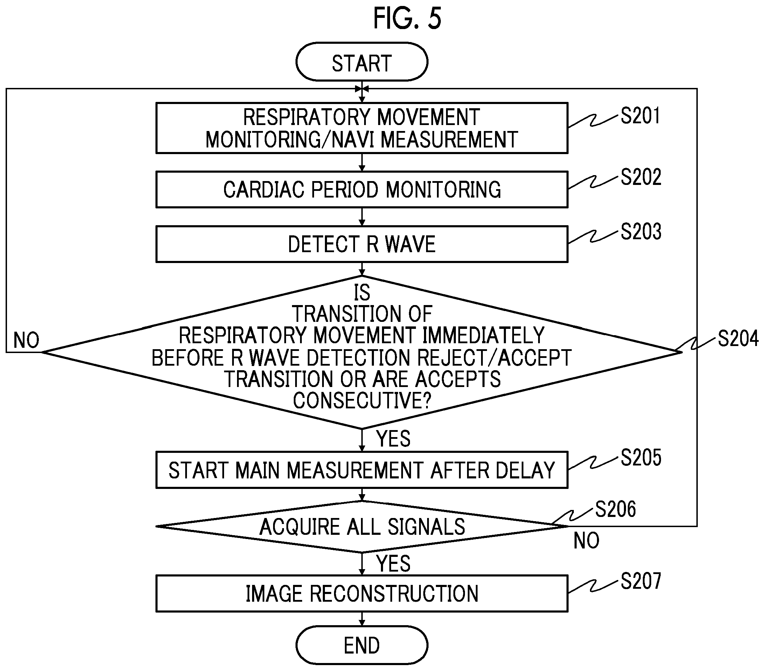

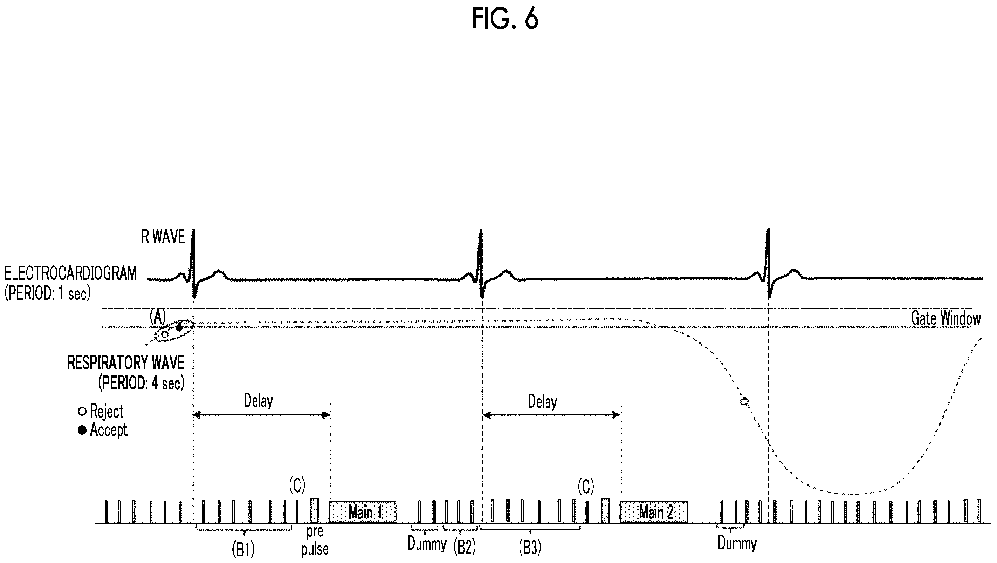

With the above process, the pre-measurements and the gating parameter setting are completed, and the imaging is started (S109). As shown in Fig. 3, the imaging includes respiratory movement monitoring (navigation measurement) S3, cardiac period monitoring S4, and the main measurement S5. Hereinafter, a flow of control will be described with reference to Figs. 5 and 6. Fig. 5 shows a flow of control, and Fig. 6 shows the cardiac period, the respiratory period, and the progress of the measurement along a time axis. Fig. 6 shows, as an example, the electrocardiogram waveform, the respiratory movement, and the elapse of the time of the measurement in a case in which two main measurements are performed in two cardiac periods to acquire one data. In Fig. 6, an accept signal generated by the respiratory movement monitoring unit 213 is indicated by "•", and a reject signal is indicated by "∘".

In a case in which the imaging is started, the measurement controller 21 starts the navigation measurement, and detects the displacement of the diaphragm due to the respiratory movement (S201). Specifically, the imaging unit 10 acquires the nuclear magnetic resonance signal (data) from an elongated region including the diaphragm in a substantially body axis direction, and the respiratory movement monitoring unit 213 detects the position of the diaphragm from the image obtained by performing one-dimensional Fourier transformation on the data of the elongated region in a direction parallel to the body axis direction, and displays the position of the diaphragm on the UI unit 30 (display device). The user looks at a respiratory state of the subject after the start of imaging, and adjusts the position of the gate window set in S106 as necessary. In general, the upper end of the window is matched with the upper end of the diaphragm that moves up and down due to respiratory movement.

The cardiac period monitoring unit 215 takes in the signal from the electrocardiograph 50 before and after the respiratory movement monitoring, and transmits the gate signal to the measurement controller 21 in a case in which the R wave is detected (S203). In a case in which the gate signal is received, the measurement controller 21 confirms, from the signal received by the respiratory movement monitoring unit 213, whether the most recent transition of the signal is the transition from the reject signal to the accept signal (reject/accept transition) or the transition from the accept signal to the reject signal (accept/reject transition) (S204). As a result, as shown in Fig. 6, in a case in which the reject/accept transition is confirmed at the timing shown in (A), the imaging unit 10 is controlled to start the main measurement (Main1) after the predetermined delay time from the R wave (S205). The delay time from the R wave to the start of the main measurement is the time set in the gating parameter setting step (Fig. 3: S2 and Fig. 4: S106). It should be noted that, unlike the example shown in Fig. 6, the same process is performed even in a case in which two or more consecutive accepts are confirmed at the timing shown in (A) of Fig. 6, instead of the reject/accept transition.

Further, as shown in Fig. 6, the navigation measurement (B1) is continued for the delay time, and the respiratory movement position is monitored. Then, it is determined whether or not the correction of the slice position of the following main measurement is necessary by using the position of the respiratory movement detected by the navi echo C acquired immediately before the elapse of the delay time. For example, the main measurement is performed by changing the slice position by an amount of shift corresponding to a difference ΔD between the position of the respiratory movement detected by the navi echo C and a predetermined position (reference position) in the gate window, or ΔD × coefficient. As the coefficient, for example, a value obtained by experience as a ratio of a displacement width of the heart to a displacement width of the diaphragm can be used, and is usually set to about 60%. In a case in which the difference ΔD between the position of the respiratory movement and the reference position is zero, the main measurement is started without adjusting the slice position.

The adjustment of the slice position is performed by adjusting the frequency of the excitation RF. By performing such a slice position correction, the misregistration can be prevented.

The main measurement is not particularly limited, but in a case of the heart imaging, "Black Blood" in which a blood signal in the heart is suppressed and a diagnosis image of a heart wall shape is acquired, or a coronary artery examination called Whole Heart Coronary Angiography (WHCA) is executed. In these types of heart imaging, the pulse sequence of the main measurement is executed after the application of the pre-pulse. Fig. 6 shows such a pre-pulse before the main measurement (Main1, Main2). Specifically, in "Black Blood", the entire left ventricle is imaged in the short axis cross section determined in S104 in Fig. 4 by a 2D-fast spin echo (FSE) method sequence after applying an inversion pulse (IR) twice in the order of slice non-selection and slice selection as the pre-pulse. In addition, in "WHCA", a fat suppression pulse (FatSat) is applied as the pre-pulse, and the entire heart is imaged in the Ax cross section by using, for example, a 3D-balanced steady-state acquisition with rewinded gradient echo (SARGE) (BASG) sequence.

After the end of the main measurement, the navigation measurement is further performed to monitor the respiratory movement. It should be noted that the navi echo generated after the end of the main measurement in Fig. 6 is a dummy that is not used for detecting the respiratory movement displacement until the signal is stabilized, and the respiratory movement monitoring unit 213 continues the respiratory movement monitoring by using the navi echo (B2) after the signal is stabilized. The accuracy of the respiratory movement monitoring can be improved by providing the dummy.

In the example shown in Fig. 6, at the second R wave detection time, the respiratory movement is in the stable period and the accept signal is emitted, so that the most recent transition of the respiratory movement signal of this R wave detection is the reject/accept transition, as in the first R wave detection. Therefore, even after the second R wave detection, the main measurement (Main2) is performed after the predetermined delay. The main measurement is the same as the first main measurement (Main1) in that the slice position is adjusted by reflecting the information on the respiratory movement displacement obtained by the navi echo C immediately before the main measurement.

On the other hand, in a case in which the transition of the respiratory movement before the R wave detection is not the reject/accept transition (S204), the respiratory movement monitoring state (S201) is restored without performing the main measurement, and the main measurement is in a standby state until the next reject/accept transition. In the example shown in Fig. 6, at the third R wave detection, since the most recent transition of the signal is the accept/reject transition and the reject signal is emitted, the main measurement is not performed, and only the navigation measurement is continued (from S204 to S201).

The above process is repeated, and after collecting the image data to be collected in the main measurement, the process proceeds to image reconstruction (S206 and S207). It should be noted that, as described above, there are several methods in the imaging method, and the measurement by one or more imaging methods may be performed in one imaging. The "main measurement" of the present embodiment includes such a case.

As described above, in the present embodiment, the navi for monitoring the respiratory movement is consecutively executed, and the start of the respiratory stable period is detected. In a case in which the electrocardiogram wave is detected, the navi is continuously executed until a desired cardiac time phase is reached in a state of ensuring that the respiratory movement is in the stable period, and the main measurement is performed. As a result, the data can be acquired without missing the timing at which the cardiac time phase and the respiratory time phase (displacement) are matched with each other. In addition, in a case in which the respiratory stable period is continued until the next cardiac period, the data acquisition can be consecutively performed, and the imaging efficiency is improved.

Further, according to the present embodiment, since the image data is acquired by correcting the imaging slice position corresponding to the displacement by using the respiratory displacement detected by the navi immediately before the data acquisition timing is the desired cardiac time phase, it is possible to obtain the image without the misregistration.

### Embodiment 2

In Embodiment 1, the subject whose respiratory movement is relatively stable is a target. On the other hand, in the present embodiment 2 according to the invention, the subject whose respiratory stable period is relatively short is a target.

In a case in which the respiratory stable period is short, there is a possibility of receiving the reject signal between the navigation measurements B2 and B3 shown in Fig. 6, that is, between the end of one main measurement and the start of the next main measurement. In this case, the second main measurement cannot be performed, so that the imaging efficiency is decreased. In the present embodiment, the gate window is set slightly below an apex (expiration) of the respiratory waveform in order to avoid a significant decrease in efficiency.

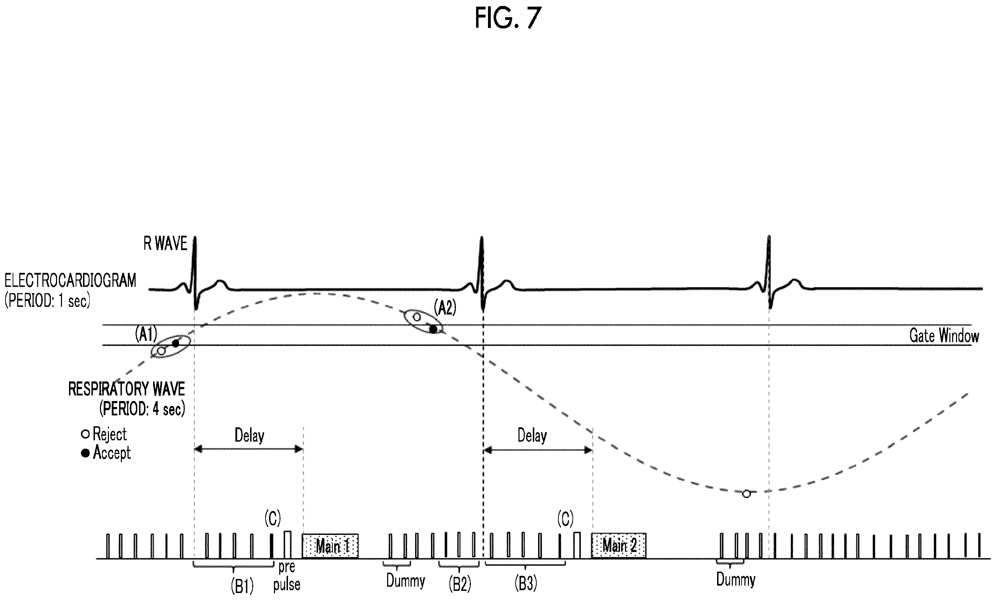

By shifting the position of the gate window downward in this way, as shown in Fig. 7, according to the invention, the transition from the reject signal to the accept signal occurs once before and after the apex of the respiratory waveform, respectively, and the main measurement is performed after the reject/accept transition, so that the imaging for performing the main measurement (acquisition of one image data) twice in two cardiac periods can be performed.

Even in the present embodiment, the flow of the control is the same as the flow shown in Fig. 5, and the respiratory movement monitoring is performed together with the start of the imaging, and the main measurement is performed using the R wave detection as the gate signal after the accept signal is emitted, as in Embodiment 1. The control of the present embodiment will be described with reference to Fig. 7.

The navigation measurement is started, and in a case in which the respiratory movement enters the inside of the gate window, the signal from the respiratory movement monitoring unit 213 is changed from the reject signal to the accept signal. In a case in which the R wave is detected in this state, the main measurement (Main1) is set to be started after the predetermined delay. The navigation measurement is performed until the start of the main measurement. Since the gate window is set at the position lower than the apex of the respiratory movement, the gate window is displaced to the outside of the gate window (accept/reject transition) while the navigation measurement is performed from B1 to the main measurement, but the gate window is in the stable period. However, here as well, the slice position correction corresponding to the displacement of the respiratory movement detected by the navi echo C immediately before the main measurement is performed, and the main measurement is executed.

In the present embodiment, since the stable period is short and the gate window is set below the apex, the signal from the respiratory movement monitoring unit is changed from the reject signal to the accept signal again by the time of the next R wave. In a case in which the second R wave is detected in this state, the main measurement (Main2) is set to be started after the predetermined delay. Here, the respiratory movement is transitioned to a state in which the reject signal is emitted between the delay period and the navigation measurement, but the slice position correction corresponding to the displacement of the respiratory movement detected by the navi echo C immediately before the main measurement is performed, and the main measurement is executed, whereby it is possible to perform the imaging of acquiring one image data with two heartbeats while suppressing the influence of the body movement. Then, the navigation measurement is consecutively executed until the reject signal is changed to the accept signal, and the main measurement is not performed during this period. It should be noted that, in the periods shown in (A1) and (A2) of the example of Fig. 7, the same process is performed even in a case in which two or more consecutive accepts are confirmed instead of the reject/accept transition.

According to the present embodiment, even in a case in which the respiratory stable period is short, the data can be acquired twice in one respiratory period, and the decrease in the imaging efficiency can be prevented. In addition, since the slice position of the main measurement is adjusted by using the displacement information obtained by the navi echo immediately before the main measurement, it is possible to acquire the image without the misregistration even in a case in which the stable period is short.

Although the embodiments of the MRI apparatus and the control method of the MRI apparatus according to the embodiment of the present invention have been described with reference to the heart imaging, the present invention is not limited to the heart imaging and can be applied to imaging of a tissue that is easily affected by the pulsation and the respiratory movement, such as an artery or a tissue near the heart, and the same effect can be obtained. In addition, a case has been described in which the R wave from the electrocardiograph is used to monitor the cardiac period, but it is needless to say that, as long as the cardiac period can be monitored, a pulse wave meter or the like can be used, in addition to the electrocardiograph.

### Explanation of References

1: MRI apparatus
10: imaging unit
20: computer
21: measurement controller
211: prior information setting unit
213: respiratory movement monitoring unit
215: cardiac period monitoring unit
30: UI unit
40: storage device
50: electrocardiograph

## Claims

1. A magnetic resonance imaging apparatus (1) comprising:
an imaging unit (10) that is configured to collect a nuclear magnetic resonance signal generated from a subject (105), and perform a navigation measurement for detecting a respiratory movement waveform of the subject (105) and a main measurement for generating an image of the subject (105);
a measurement controller (21) that is configured to control the imaging unit (10);
a prior information setting unit (211) that is configured to set a gate window at a position in a stable period of the respiratory movement waveform of the subject (105);
a respiratory movement monitoring unit (213) that is configured to monitor the respiratory movement waveform of the subject (105) by using a result of the navigation measurement, and transmit an accept signal and a reject signal to the measurement controller (21) depending on whether a magnitude of the respiratory movement waveform of the subject (105) is inside or outside the preset gate window; and
a cardiac period monitoring unit (215) that is configured to monitor a cardiac period of the subject (105), and generate a gate signal at an R wave occurrence timing of the cardiac period,
wherein the measurement controller (21) is configured to consecutively execute the navigation measurement before and after the generation of the gate signal, and control, in a case in which the gate signal is received from the cardiac period monitoring unit (215), the imaging unit (10) to perform the main measurement after a preset delay time elapses in a case in which a most recent transition among transitions of a signal from the respiratory movement monitoring unit (213), which is generated during the navigation measurement before the reception of the gate signal, is a transition from the reject signal to the accept signal,
**characterised in that** the prior information setting unit (211) is configured to set an upper limit of the gate window to a value lower than a maximum value of the stable period of the respiratory movement waveform of the subject (105) so that the transition from the reject signal to the accept signal occurs once before and after an apex of the respiratory movement waveform, respectively.

2. The magnetic resonance imaging apparatus (1) according to claim 1, wherein the respiratory movement monitoring unit (213) does not use a result of a navigation measurement most recent to the main measurement among the navigation measurements performed after the main measurement, for monitoring the respiratory movement waveform of the subject (105).

3. The magnetic resonance imaging apparatus (1) according to claim 1 or 2, wherein the measurement controller (21) is configured to adjust a slice position of the main measurement following a navigation measurement performed in the delay time by using a respiratory movement position obtained by the navigation measurement.

4. The magnetic resonance imaging apparatus (1) according to any of claims 1 to 3, wherein the measurement performed by the imaging unit (10) includes scanogram imaging for determining an imaging part of the subject (105) by imaging three cross sections of the imaging part at the center of the imaging space, and the prior information setting unit (211) is configured to determine the position of the gate window by using an image obtained by the scanogram imaging.

5. The magnetic resonance imaging apparatus (1) according to any of claims 1 to 3, further comprising:
a UI unit (30) that is configured to receive adjustment of the position of the gate window by a user,
wherein the respiratory movement monitoring unit (213) is configured to reflect the adjustment received by the UI unit (30) to change the position of the gate window set by the prior information setting unit (211).

6. The magnetic resonance imaging apparatus (1) according to any of claims 1 to 3, wherein
the prior information setting unit (211) is configured to set the delay time of the main measurement,
the magnetic resonance imaging apparatus (1) is configured to obtain a cine image by cine imaging on a region including a heart of the subject (105) as a target, and
the prior information setting unit (211) is configured to receive the cine image and determine the delay time by using the cine image.

7. A control method of a magnetic resonance imaging apparatus (1) that includes an imaging unit (10) controlled by a measurement controller (21), the control method being configured to collect a nuclear magnetic resonance signal from a subject (105) for generating a subject image as a main measurement in a case in which an electrocardiogram waveform and a respiratory movement waveform of the subject (105) are in determined ranges, respectively, the control method comprising:
a step of consecutively executing a navigation measurement for detecting the respiratory movement waveform before and after the main measurement;
a step of setting a gate window at a position in a stable period of the respiratory movement waveform of the subject (105);
a step of monitoring a result of the navigation measurement, generating an accept signal in a case in which a magnitude of a respiratory movement is inside the set gate window, and generating a reject signal in a case in which the magnitude of the respiratory movement waveform is outside the gate window;
a step of monitoring a cardiac period of the subject (105) and generating a gate signal at an R wave occurrence timing of the cardiac period; and
a step of, after an R wave of the electrocardiogram waveform is detected, in a case in which a most recent transition among transitions of a signal generated during the navigation measurement before the R wave is detected is a transition from the reject signal to the accept signal, starting the main measurement after a preset delay time,
**characterised in that** an upper limit of the gate window is set to a value lower than a maximum value of the stable period of the respiratory movement waveform of the subject (105) so that the transition from the reject signal to the accept signal occurs once before and after an apex of the respiratory movement waveform, respectively.

8. The control method of a magnetic resonance imaging apparatus (1) according to claim 7, further comprising:
a step of adjusting a slice position of the main measurement by using a result of a navigation measurement performed immediately before the main measurement.

9. The control method of a magnetic resonance imaging apparatus (1) according to claim 7 or 8, further comprising:
a step of performing scanogram imaging by imaging three cross sections of the imaging part at the center of the imaging space; and
a step of acquiring information on the respiratory movement of the subject (105) from a result of the scanogram imaging, and setting the position of the gate window.

10. The control method of a magnetic resonance imaging apparatus (1) according to any of claims 7 to 9, further comprising:
a step of acquiring a cine image of a heart; and
a step of acquiring information on a cardiac period from the cine image, and determining the delay time.

## Patentansprüche

1. Magnetresonanztomographie-Vorrichtung (1), umfassend:
eine Bildgebungseinheit (10), die so konfiguriert ist, dass sie ein von einer Untersuchungsperson (105) erzeugtes Kernmagnetresonanz-Signal sammelt und eine Navigationsmessung zum Detektieren einer Atembewegungs-Wellenform der Untersuchungsperson (105) und eine Hauptmessung zum Erzeugen eines Bildes der Untersuchungsperson (105) durchführt;
eine Messsteuerung (21), die so konfiguriert ist, dass sie die Bildgebungseinheit (10) steuert;
eine Vorinformations-Einstelleinheit (211), die so konfiguriert ist, dass sie ein Gate-Fenster an einer Position in einer stabilen Periode der Atembewegungs-Wellenform der Untersuchungsperson (105) einstellt;
eine Atembewegungs-Überwachungseinheit (213), die so konfiguriert ist, dass sie die Atembewegungs-Wellenform der Untersuchungsperson (105) unter Verwendung eines Ergebnisses der Navigationsmessung überwacht und ein Akzeptanzsignal und ein Zurückweisungssignal an die Messsteuerung (21) in Abhängigkeit davon überträgt, ob eine Größe der Atembewegungs-Wellenform der Untersuchungsperson (105) innerhalb oder außerhalb des voreingestellten Gate-Fensters liegt; und
eine Herzperioden-Überwachungseinheit (215), die so konfiguriert ist, dass sie eine Herzperiode der Untersuchungsperson (105) überwacht und ein Gate-Signal zu einem R-Wellen-Auftretenszeitpunkt der Herzperiode erzeugt,
wobei die Messsteuerung (21) so konfiguriert ist, dass sie die Navigationsmessung nacheinander vor und nach der Erzeugung des Gate-Signals ausführt und in einem Fall, in dem das Gate-Signal von der Herzperioden-Überwachungseinheit (215) empfangen wird, die Bildgebungseinheit (10) so steuert, dass sie die Hauptmessung nach Ablauf einer voreingestellten Verzögerungszeit in einem Fall durchführt, in dem ein jüngster Übergang unter Übergängen eines Signals von der Atembewegungs-Überwachungseinheit (213), das während der Navigationsmessung vor dem Empfang des Gate-Signals erzeugt wird, ein Übergang von dem Zurückweisungssignal zu dem Akzeptanzsignal ist,
**dadurch gekennzeichnet, dass** die Vorinformations-Einstelleinheit (211) so konfiguriert ist, dass sie eine Obergrenze des Gate-Fensters auf einen Wert, der niedriger als ein Maximalwert der stabilen Periode der Atembewegungs-Wellenform der Untersuchungsperson (105) ist, einstellt, so dass der Übergang von dem Zurückweisungssignal zu dem Akzeptanzsignal jeweils einmal vor und nach einem Scheitelpunkt der Atembewegungs-Wellenform auftritt.

2. Magnetresonanztomographie-Vorrichtung (1) nach Anspruch 1,
wobei die Atembewegungs-Überwachungseinheit (213) ein Ergebnis einer Navigationsmessung, die unter den Navigationsmessungen, die nach der Hauptmessung durchgeführt werden, der Hauptmessung am nächsten ist, nicht zum Überwachen der Atembewegungs-Wellenform der Untersuchungsperson (105) verwendet.

3. Magnetresonanztomographie-Vorrichtung (1) nach Anspruch 1 oder 2,
wobei die Messsteuerung (21) so konfiguriert ist, dass sie eine Schichtposition der Hauptmessung, die einer in der Verzögerungszeit durchgeführten Navigationsmessung folgt, unter Verwendung einer durch die Navigationsmessung erhaltenen Atembewegungsposition anpasst.

4. Magnetresonanztomographie-Vorrichtung (1) nach einem der Ansprüche 1 bis 3,
wobei die von der Bildgebungseinheit (10) durchgeführte Messung Scanogramm-Bildgebung zum Bestimmen eines Bildgebungsteils der Untersuchungsperson (105) durch Bildgebung von drei Querschnitten des Bildgebungsteils in der Mitte des Bildgebungsraums enthält und die Vorinformations-Einstelleinheit (211) so konfiguriert ist, dass sie die Position des Gate-Fensters unter Verwendung eines durch die Scanogramm-Bildgebung erhaltenen Bildes bestimmt.

5. Magnetresonanztomographie-Vorrichtung (1) nach einem der Ansprüche 1 bis 3, ferner umfassend:
eine UI-Einheit (30), die so konfiguriert ist, dass sie Anpassung der Position des Gate-Fensters durch einen Benutzer empfängt,
wobei die Atembewegungs-Überwachungseinheit (213) so konfiguriert ist, dass sie die von der UI-Einheit (30) empfangene Anpassung widerspiegelt, um die Position des von der Vorinformations-Einstelleinheit (211) eingestellten Gate-Fensters zu ändern.

6. Magnetresonanztomographie-Vorrichtung (1) nach einem der Ansprüche 1 bis 3, wobei
die Vorinformations-Einstelleinheit (211) so konfiguriert ist, dass sie die Verzögerungszeit der Hauptmessung einstellt,
die Magnetresonanztomographie-Vorrichtung (1) so konfiguriert ist, dass sie ein Cine-Bild durch Cine-Bildgebung an einem Bereich, der ein Herz der Untersuchungsperson (105) enthält, als ein Ziel erhält, und
die Vorinformations-Einstelleinheit (211) so konfiguriert ist, dass sie das Cine-Bild empfängt und die Verzögerungszeit unter Verwendung des Cine-Bildes bestimmt.

7. Steuerverfahren einer Magnetresonanztomographie-Vorrichtung (1), die eine Bildgebungseinheit (10) enthält, die von einer Messsteuerung (21) gesteuert wird, wobei das Steuerverfahren so konfiguriert ist, dass es ein Kernmagnetresonanz-Signal von einer Untersuchungsperson (105) zum Erzeugen eines Motivbildes als eine Hauptmessung in einem Fall sammelt, in dem eine Elektrokardiogramm-Wellenform und eine Atembewegungs-Wellenform der Untersuchungsperson (105) jeweils in bestimmten Bereichen liegen, wobei das Steuerverfahren umfasst:
einen Schritt des Ausführens einer Navigationsmessung nacheinander zum Detektieren der Atembewegungs-Wellenform vor und nach der Hauptmessung;
einen Schritt des Einstellens eines Gate-Fensters an einer Position in einer stabilen Periode der Atembewegungs-Wellenform der Untersuchungsperson (105);
einen Schritt des Überwachens eines Ergebnisses der Navigationsmessung, des Erzeugens eines Akzeptanzsignals in einem Fall, in dem eine Größe einer Atembewegung innerhalb des eingestellten Gate-Fensters liegt, und des Erzeugens eines Zurückweisungssignals in einem Fall, in dem die Größe der Atembewegungs-Wellenform außerhalb des Gate-Fensters liegt;
einen Schritt des Überwachens einer Herzperiode der Untersuchungsperson (105) und des Erzeugens eines Gate-Signals zu einem R-Wellen-Auftretenszeitpunkt der Herzperiode; und
einen Schritt des Startens der Hauptmessung nach einer voreingestellten Verzögerungszeit, nachdem eine R-Welle der Elektrokardiogramm-Wellenform detektiert wird, in einem Fall, in dem ein jüngster Übergang unter Übergängen eines Signals, das während der Navigationsmessung erzeugt wird, bevor die R-Welle detektiert wird, ein Übergang von dem Zurückweisungssignal zu dem Akzeptanzsignal ist,
**dadurch gekennzeichnet, dass** eine Obergrenze des Gate-Fensters auf einen Wert, der niedriger als ein Maximalwert der stabilen Periode der Atembewegungs-Wellenform der Untersuchungsperson (105) ist, eingestellt wird, so dass der Übergang von dem Zurückweisungssignal zu dem Akzeptanzsignal jeweils einmal vor und nach einem Scheitelpunkt der Atembewegungs-Wellenform auftritt.

8. Steuerverfahren einer Magnetresonanztomographie-Vorrichtung (1) nach Anspruch 7, ferner umfassend:
einen Schritt des Anpassens einer Schichtposition der Hauptmessung unter Verwendung eines Ergebnisses einer Navigationsmessung, die unmittelbar vor der Hauptmessung durchgeführt wird.

9. Steuerverfahren einer Magnetresonanztomographie-Vorrichtung (1) nach Anspruch 7 oder 8, ferner umfassend:
einen Schritt des Durchführens von Scanogramm-Bildgebung durch Bildgebung von drei Querschnitten des Bildgebungsteils in der Mitte des Bildgebungsraums; und
einen Schritt des Erfassens von Informationen über die Atembewegung der Untersuchungsperson (105) aus einem Ergebnis der Scanogramm-Bildgebung und des Einstellens der Position des Gate-Fensters.

10. Steuerverfahren einer Magnetresonanztomographie-Vorrichtung (1) nach einem der Ansprüche 7 bis 9, ferner umfassend:
einen Schritt des Erfassens eines Cine-Bildes eines Herzens; und
einen Schritt des Erfassens von Informationen über eine Herzperiode aus dem Cine-Bild und des Bestimmens der Verzögerungszeit.

## Revendications

1. Appareil d'imagerie par résonance magnétique (1) comprenant :
une unité d'imagerie (10) qui est configurée pour acquérir un signal de résonance magnétique nucléaire généré à partir d'un sujet (105), et pour effectuer une mesure de navigation pour détecter une forme d'onde de mouvement respiratoire du sujet (105) et une mesure principale pour générer une image du sujet (105) ;
un contrôleur de mesure (21) qui est configuré pour contrôler l'unité d'imagerie (10) ;
une unité de réglage des informations préalables (211) qui est configurée pour régler une fenêtre de synchronisation à une position dans une période stable de la forme d'onde de mouvement respiratoire du sujet (105) ;
une unité de surveillance de mouvement respiratoire (213) qui est configurée pour surveiller la forme d'onde de mouvement respiratoire du sujet (105) en utilisant un résultat de la mesure de navigation, et pour transmettre un signal d'acceptation et un signal de rejet au contrôleur de mesure (21) selon qu'une magnitude de la forme d'onde de mouvement respiratoire du sujet (105) se situe à l'intérieur ou à l'extérieur de la fenêtre de synchronisation prédéfinie ; et
une unité de surveillance de période cardiaque (215) qui est configurée pour surveiller une période cardiaque du sujet (105), et pour générer un signal de synchronisation à un moment de l'apparition de l'onde R de la période cardiaque,
dans lequel le contrôleur de mesure (21) est configuré pour exécuter consécutivement la mesure de navigation avant et après la génération du signal de synchronisation, et pour contrôler, dans un cas où le signal de synchronisation est reçu de l'unité de surveillance de période cardiaque (215), l'unité d'imagerie (10) pour effectuer la mesure principale après l'écoulement d'un temps de retard prédéfini dans un cas où une transition la plus récente, parmi des transitions d'un signal provenant de l'unité de surveillance de mouvement respiratoire (213), qui est généré pendant la mesure de navigation avant la réception du signal de synchronisation, est une transition du signal de rejet vers le signal d'acceptation,
**caractérisé en ce que** l'unité de réglage des informations préalables (211) est configurée pour régler une limite supérieure de la fenêtre de synchronisation à une valeur inférieure à une valeur maximale de la période stable de la forme d'onde de mouvement respiratoire du sujet (105) de sorte que la transition du signal de rejet vers le signal d'acceptation se produise une fois respectivement avant et après un sommet de la forme d'onde de mouvement respiratoire.

2. Appareil d'imagerie par résonance magnétique (1) selon la revendication 1,
dans lequel l'unité de surveillance de mouvement respiratoire (213) n'utilise pas un résultat d'une mesure de navigation la plus récente par rapport à la mesure principale, parmi les mesures de navigation effectuées après la mesure principale, pour surveiller la forme d'onde de mouvement respiratoire du sujet (105).

3. Appareil d'imagerie par résonance magnétique (1) selon la revendication 1 ou la revendication 2,
dans lequel le contrôleur de mesure (21) est configuré pour ajuster une position de coupe de la mesure principale suivant une mesure de navigation effectuée pendant le temps de retard en utilisant une position de mouvement respiratoire obtenue par la mesure de navigation.

4. Appareil d'imagerie par résonance magnétique (1) selon l'une quelconque des revendications 1 à 3,
dans lequel la mesure effectuée par l'unité d'imagerie (10) inclut une imagerie de scanogramme pour déterminer une partie à imager du sujet (105) par imagerie de trois sections transversales de la partie à imager au centre de l'espace d'imagerie, et l'unité de réglage des informations préalables (211) est configurée pour déterminer la position de la fenêtre de synchronisation en utilisant une image obtenue par l'imagerie de scanogramme.

5. Appareil d'imagerie par résonance magnétique (1) selon l'une quelconque des revendications 1 à 3, comprenant en outre :
une unité d'interface utilisateur (30) qui est configurée pour recevoir un ajustement de la position de la fenêtre de synchronisation par un utilisateur,
dans lequel l'unité de surveillance de mouvement respiratoire (213) est configurée pour réfléchir l'ajustement reçu par l'unité d'interface utilisateur (30) pour modifier la position de la fenêtre de synchronisation réglée par l'unité de réglage des informations préalables (211).

6. Appareil d'imagerie par résonance magnétique (1) selon l'une quelconque des revendications 1 à 3, dans lequel
l'unité de réglage des informations préalables (211) est configurée pour régler le temps de retard de la mesure principale,
l'appareil d'imagerie par résonance magnétique (1) est configuré pour obtenir une image ciné par imagerie ciné sur une région incluant un cœur du sujet (105) comme cible, et
l'unité de réglage des informations préalables (211) est configurée pour recevoir l'image ciné et déterminer le temps de retard en utilisant l'image ciné.

7. Procédé de contrôle d'un appareil d'imagerie par résonance magnétique (1) qui inclut une unité d'imagerie (10) contrôlée par un contrôleur de mesure (21), le procédé de contrôle étant configuré pour acquérir un signal de résonance magnétique nucléaire d'un sujet (105) pour générer une image de sujet comme mesure principale dans un cas où une forme d'onde d'électrocardiogramme et une forme d'onde de mouvement respiratoire du sujet (105) se trouvent respectivement dans des plages déterminées, le procédé de contrôle comprenant :
une étape consistant à effectuer consécutivement une mesure de navigation pour détecter la forme d'onde de mouvement respiratoire avant et après la mesure principale ;
une étape consistant à régler une fenêtre de synchronisation à une position dans une période stable de la forme d'onde de mouvement respiratoire du sujet (105) ;
une étape consistant à surveiller un résultat de la mesure de navigation, à générer un signal d'acceptation dans un cas où une magnitude d'un mouvement respiratoire se situe à l'intérieur de la fenêtre de synchronisation réglée, et à générer un signal de rejet dans un cas où la magnitude de la forme d'onde de mouvement respiratoire se situe à l'extérieur de la fenêtre de synchronisation ;
une étape consistant à surveiller une période cardiaque du sujet (105) et à générer un signal de synchronisation à un moment de l'apparition de l'onde R de la période cardiaque ; et
une étape consistant, après la détection d'une onde R de la forme d'onde d'électrocardiogramme, dans un cas où une transition la plus récente, parmi des transitions d'un signal généré pendant la mesure de navigation avant la détection de l'onde R, est une transition du signal de rejet vers le signal d'acceptation, à démarrer la mesure principale après un temps de retard prédéfini,
**caractérisé en ce qu'**une limite supérieure de la fenêtre de synchronisation est réglée à une valeur inférieure à une valeur maximale de la période stable de la forme d'onde de mouvement respiratoire du sujet (105) de sorte que la transition du signal de rejet vers le signal d'acceptation se produise une fois respectivement avant et après un sommet de la forme d'onde de mouvement respiratoire.

8. Procédé de contrôle d'un appareil d'imagerie par résonance magnétique (1) selon la revendication 7, comprenant en outre :
une étape consistant à ajuster une position de coupe de la mesure principale en utilisant un résultat d'une mesure de navigation effectuée immédiatement avant la mesure principale.

9. Procédé de contrôle d'un appareil d'imagerie par résonance magnétique (1) selon la revendication 7 ou la revendication 8, comprenant en outre :
une étape consistant à effectuer une imagerie de scanogramme par imagerie de trois sections transversales de la partie à imager au centre de l'espace d'imagerie ; et
une étape étape consistant à acquérir des informations sur le mouvement respiratoire du sujet (105) à partir d'un résultat de l'imagerie de scanogramme, et à régler la position de la fenêtre de synchronisation.

10. Procédé de contrôle d'un appareil d'imagerie par résonance magnétique (1) selon l'une quelconque des revendications 7 à 9, comprenant en outre :
une étape consistant à acquérir une image ciné d'un cœur ; et
une étape consistant à acquérir des informations sur une période cardiaque à partir de l'image ciné, et à déterminer le temps de retard.
